# EUROPEAN PATENT APPLICATION

(11) **EP 4 620 337 A1**
(43) Date of publication of application: **24.09.2025**
(21) Application number: 24164932.6
(22) Date of filing: 20.03.2024
(51) Int. Cl.: A41C 1/10, A61F 13/49

(54) **POST-NATAL ABSORBENT HYGIENE SLIP**

(71) Applicant: Corman SpA, 20084 Lacchiarella (IT)
(72) Inventor: MANTOVANI, Giorgio, 20084 Lacchiarella (IT)
(74) Representative: Wächtershäuser & Hartz Patentanwaltspartnerschaft mbB

(57) **Abstract**

A post-natal absorbent hygiene slip, comprising a chassis (1) comprising a front panel and a back panel and multiple elastic elements including waist elastics (3), leg elastics (4), elastics in the front and/or back panels of the slip (5), and leg cuff elastics (6),
wherein said slip further comprises one or more supportive elastic element (11) at least in the front panel of the slip, said one or more supportive elastic element (11) being attached to the chassis (i) at first and second waist suspension points (13, 15) in side-seam areas of the slip not more than 2 cm from the waist edge (19) and (ii) at a lower suspension point (17) being distanced from the waist edge (19) of the slip by at least length L/4 of the length (L) of the open form of the slip.

## Description

### FIELD OF THE INVENTION

The present invention relates to a hygiene slip for women post-partum (post-natal). The slip helps preventing or reducing the consequences of stretching and weakening of the abdominal muscles and skin stretching of women during pregnancy, such as sagging belly. The slip comprises an absorbent core designed to safely absorb lochia, a vaginal discharge after giving birth. The invention also relates to a manufacturing process for producing the slip.

### BACKGROUND OF THE INVENTION

During pregnancy, growth of the abdomen involves stretching and weakening of the straight and oblique abdominal muscles. After delivery of a newborn, the stretched and weakened abdominal muscles and skin generally remain stretched, leading to a sagging belly. Moreover, during the weeks after delivery, different types of fluids known as lochia and containing a mix of blood, mucus and uterine tissue are released that include changing amounts of solid or semi-solid matter such as blood clots.

Known solutions to handle these problems are the use of hygienic slips or incontinence slips for the latter problem and compression underwear and/or elasticated belly bandages for the former problem.

US5897423 A describes a post-pregnancy compression garment that provides abdominal support to facilitate a faster return to the individual's pre-pregnancy body shape. The garment consists of a tubular body encompassing an anatomical region from the thighs to beneath the chest. Compression material or strips are constructed from an elastic memory fabric positioned so as to cause compression to the abdominal area. Single use absorbent slips are described in a number of patents, e.g. US 9,592,163 or US20090264847. JP7195945 B2 describes an incontinence slip with curved elastics supporting safe position when climbing stairs. The prior art is, however, insufficient in that it provides insufficient effect in preventing or reducing the sagging belly problem and, at the same time, handling of the different types of lochia released post partum.

Departing from the prior art, it is a problem of the invention to provide a hygiene slip that can reduce the consequences of stretching and weakening of the abdominal muscles and skin of women during pregnancy. The slip should at the same time allow absorbing lochia.

### SUMMARY OF THE INVENTION

This problem is solved by:
1) a post-natal absorbent hygiene slip, comprising a front panel and a back panel and multiple elastic elements including waist elastics (3), leg elastics (4), elastics in the front and/or back panels of the slip (5), and leg cuff elastics (6),
   wherein said slip further comprises one or more supportive elastic element (11) at least in the front panel of the slip, said one or more supportive elastic element (11) being attached to the slip:
   (i) at first and second waist suspension points (13, 15) in side-seam areas of the slip, said waist suspension points being not more than 2 cm from the waist edge (19) and
   (ii) at a lower suspension point (17) being distanced from the waist edge (19) of the slip by at least length L/4 of the length L of the open form of the slip in longitudinal direction and positioned in the middle in cross direction of the slip.
2) The slip according to item 1, wherein said supportive elastic element(s) (11) connects said waist suspension points (13, 15) with said lower suspension point (17) along a trough-shaped curve.
3) The slip according to item 2, wherein said trough-shaped elastic elements or said slip comprises additional supportive elastic elements (12) positioned above and/or below said supportive elastic element (11).
4) The slip according to any one of items 1 to 3, comprising an absorbent core (2).
5) The slip according to any one of items 1 to 4, comprising a topsheet (23) and a backsheet (25).
6) The slip according to item 5, wherein the topsheet has, at least in the area covering the absorbent core (2), openings larger than 0.5 mm², preferably larger than 1 mm², more preferably about 2 mm².
7) The slip according to any one of items 1 to 6, wherein said waist suspension points (13, 15) are located at most 2 cm from the right (31, 32) and left (33, 34) seam edges, respectively.
8) A manufacturing process for producing the slip according to any of items 1 to 7, characterized in that application of said supportive elastic element (11), preferably in a trough-shaped curve, is performed before the leg openings are provided.

The post-natal absorbent hygiene slip of the invention comprises elastic elements that extend both in cross-direction as well as in oblique direction and thus support regression of stretched abdominal tissue and muscles post-partum. In order to provide this function, the supportive elastic element(s) (11) is/are fixed to the chassis of the slip (or to the slip) in side-seam areas, close to the waist edge, of the front panel of the slip, notably close to or in the area of the horizontal waist elastics, and at a lower suspension point (17) remote from the waist edge (19). Thus, the supportive elastic element(s) is/are capable of pulling stretched tissue in the oblique upward and lateral direction, when the slip is worn, to support in particular regression of normal oblique abdominal muscles. The slip has an absorbent core and a topsheet that can be chosen accordingly to the post-partum phase of a woman.

### BRIEF DESCRIPTION OF THE FIGURES

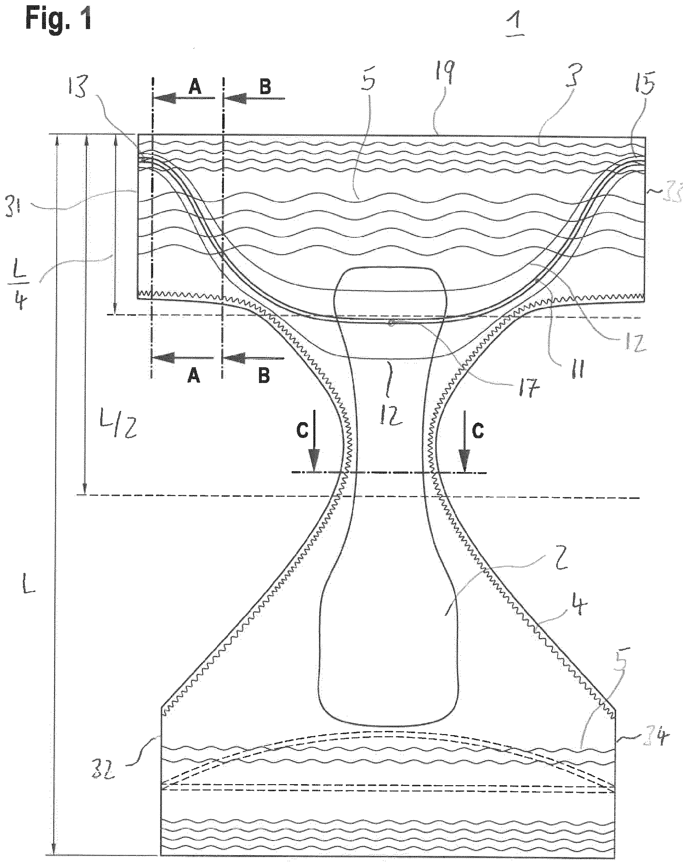

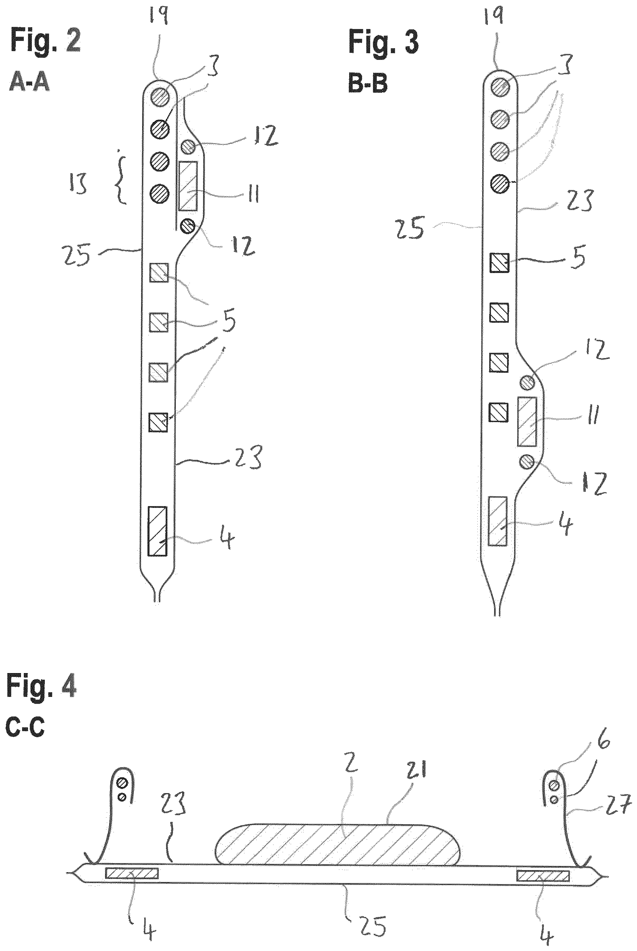

**Fig. 1** is a top view on an open form of the slip of the invention, i.e. in a state before connecting the right seam edges (31, 32) to each other and the left seam edges (33, 34) to each other to form the slip. The top half of the open form shown in Fig. 1 is the front panel, the lower is the back panel. The open form shown is deformed to a flat shape, as obtainable by pressing the open form on a flat surface against the force of elastics present therein. Dashed lines A-A, B-B, and C-C indicate the positions of the cross-sections shown in Figures 2, 3, and 4, respectively. Absorbent core (2) is covered by a topsheet (not visible in the top view Fig. 1). The presence of the absorbent core can be seen in the top view by way of its higher thickness than surrounding areas of the slip or the open form.
**Fig. 2** Cross-section along line A-A in viewing direction indicated by arrows in Fig. 1. Depicted are topsheet (23), backsheet (25), and the following elastic elements (indicated by hatched circles and rectangles): waist elastics (3), leg elastics (4), and elastics in the front panel of the slip (5). The supportive elastic element (11) is also shown, together with optional additional supportive elastic elements (12).
**Fig. 3** Cross-section along line B-B in viewing direction indicated by arrows in Fig. 1. Numerals are as in Fig. 2. See figure legend of Fig. 2.
**Fig. 4** Cross-section along line C-C in viewing direction indicated by arrows in Fig. 1. The figure shows the leg cuffs (27) containing elastic elements (6), absorbent core (2), topsheet (23), separate topsheet (21) of the absorbent core (2) and backsheet (25).

### DETAILED DESCRIPTION OF THE INVENTION

The hygiene slip of the invention generally comprises a main body or chassis (1) with elastic elements applied to it and an absorbent core (2) fixed to the chassis. The chassis of the slip, and the slip itself, comprises a front panel (upper half of the open form in Fig. 1) and a back panel (lower half of the open form in Fig. 1). The front panel is the half or part that, when the slip is worn, covers the abdomen of the wearer from the crotch area up to the waist (or waist line) of the wearer. The back panel is the half or part that covers the buttocks of the wearer up to the waist (or waist line) of the wearer. The hygiene slip of the invention is produced from the open form shown in Fig. 1 by connecting front and back panels, notably by connecting seam areas (31) and (32) as well as seam areas (33) and (34).

The chassis comprises essentially the front and back panels, both formed of backsheet(s) and topsheet(s). Different general designs of hygiene slips are known. In some of them, the chassis comprises a topsheet and a backsheet, and the absorbent core is positioned and fixed between these two layers, i.e. enveloped between these layers. In another design, the topsheet and the backsheet are connected to each other to form the chassis, while the absorbent core is positioned on and fixed to the topsheet, i.e. the absorbent core is located on the topsheet. In this design, the absorbent core may comprise an additional topsheet covering the absorbent core. In these both designs, the chassis of the slip generally consist of topsheet and backsheet, each made of one piece of material and cut in a shape to the form of slip.

In a further design, the chassis is divided into a front and rear panel part and the absorbent core having its own topsheet and backsheet connect these panel parts to form the open form of the chassis.

In a still further design, the absorbent core comprises the topsheet and the backsheet to form the crotch part of the open form, and side panels are attached to the lateral edges of the crotch part to form the open form of the slip.

All these designs of the chassis and the slip are compatible with the present invention to provide the elastics used in the invention. In a preferred embodiment of the invention, the chassis is made of one piece of material for the backsheet (25) and one piece of material for the topsheet (23). In an alternative preferred embodiment, the chassis is assembled from a front part and a back part, each comprising a topsheet and a backsheet. Preferably, the open form of the slip comprises a continuous topsheet and a continuous backsheet, at least in cross direction of the product. The leg openings may be cut, in form of an oval, out from the chassis in case they are made of one piece of topsheet and one piece of a backsheet. In case the chassis is assembled from a front and a back part, the leg opening may be cut out of the front and back parts of the chassis as fragments of an oval.

The open form of the slip and the slip itself define a cross-direction that runs along waist edge (19). The open form and slip define a longitudinal direction that runs along length (L) in Fig. 1, i.e. orthogonal to the cross-direction.

As is generally known, the slip comprises multiple layers or sheets, similarly as described in WO 2024/041826 (Corman SpA) for absorbent hygiene products. The slip generally comprises a topsheet (23) facing the body of the user, a backsheet (25) on the opposite side (facing outside), and an absorbent core (2). The absorbent core is generally limited to the crutch area of the slip where discharge of body fluids is to be expected, as shown in Fig. 1.

The topsheet has the main function of allowing permeation of fluid released from the body of a user through it and provides the soft, gentle contact surface to the wearer's skin. The absorbent core has the main function of absorbing and retaining the body fluid. The backsheet has the main function of preventing release (leakage) of fluid from the product on the side facing the outside (i.e., the side opposite to that facing the body of the user). Generally, the topsheet and the backsheet further function as outer physical boundary of the product, as an envelope that may (depending on the design of the chassis) enclose, between the top- and backsheet, the absorbent core. Alternatively, the topsheet may carry on its surface the absorbent core. In any event, the topsheet and backsheet together (when connected to each other) provide the physical strength to the slip required in use as well as for fixing the elastic elements described below thereto.

### Topsheet

The topsheet (23) and, if present, additional topsheet (21) of the absorbent core should be soft and gentle as it contacts the skin of the user. Moreover, at least in areas that directly or indirectly cover the absorbent core, it should allow fast acquisition of fluid from the body of the user and quickly pass the fluid on to the inner layers of the absorbent core. The topsheet should remain or feel dry after passing the fluid to the inner layers. The topsheet of the slip of the invention is fluid permeable. The fluid is generally liquid, but may contain varying amounts of solids, such as clots.

The topsheet of the absorbent product may be a nonwoven, e.g. made of fibers of a polypropylene (PP) or PP/PE fibers, carded or spunbonded, thermos-bonded or air-trough bonded. Such topsheet nonwovens are inter alia described in EP 91905693 B1, US3489148 A, US8530722, or EP2399558. Alternatively, the topsheet may be made by air-through bonding technology which provides a significant improvement towards even softer, bulkier, and skin-friendly nonwovens. Examples of such materials are described in EP1369519, in EP 2 708 623 and WO2014022988. A method for producing such high loft nonwovens is described in EP3246444.

Preferably, the topsheet is made of a material that is bio-based and biodegradable. The topsheet preferably is or comprises a sheet that is a fabric made of fibers of cellulose, e.g. cotton or viscose fibers, or of fibers of a cellulose derivative, e.g. fibers made of cellulose esters such as described in DE3312022 A1 and DE3246417 A1. Preferably, the fabric is a nonwoven fabric made of cellulose fibers. The nonwoven may be produced by hydroentanglement from fibers (also referred to in the art as "spunlacing"). The fibers (such as viscose fibers) used for hydroentanglement may have a median length of 5 to 30 mm, preferably from 10 to 20 mm (by number). Fiber length can be measured as described below. The median length of the fibers may be determined from the lengths of fibers of a representative sample of fibers. Suitable topsheet s are offered in the market for example by Fama Jersey S,p,A. company e.g., as Maspun 35100 C P1.

The topsheet may have perforations allowing body exudate to be quickly transferred to lower layers, notably the absorbent core. The topsheet may have, at least in the area covering the absorbent core (2), openings larger than 0,5 mm², preferably larger than 1 mm², more preferably about 2 mm². Preferably for slips provided for use in the first days post-partum for absorption of lochia rubra, the openings may have a size of from 0.5 to 10 mm², preferably of from 1.0 to 5 mm², which allows immobilizing and holding in place the solid parts of lochia, e.g. blood clots.

The topsheet may be at least partially coated with, and may thus contain a layer of, a hydrophilic polymer, preferably at least on its outer surface facing the body of the user. Examples of hydrophilic polymers are carboxymethyl cellulose, alginates or other hydrogels like gelatin, chitosan, or hyaluronic acid or a salt of the afore-mentioned polymers.

The basis weight of the topsheet nonwoven may be from 10 to 50, preferably from 20 to 50, even more preferably from 30 to 40 g/m². Its average density may be from 80 to 160, preferably from 100 to 140 kg/m³. The wicking rate of this topsheet material may be 0 measured by EDANA ERT 10.3-99. The thickness of the topsheet is generally within a range of from 0.1 to 4 mm, preferably from 0.2 to 2 mm.

The topsheet may be adjusted to the post-partum phase of the woman. For lochia rubra, the topsheet may have, at least in the area covering the absorbent core, a structure comprising folds or cavities that allow holding back blood clots present in the body exudate. For the lochia serosa, the top may comprise, at least in the area covering the absorbent core, a structure comprising fine 3-dimensional structure, such a grooves or perforations. For the lochia alba, the topsheet may be of particular softness.

### Backsheet

The backsheet (25) together with topsheet constitutes the chassis, an envelope of the absorbent product of the invention. The role of the backsheet is to provide a containment for fluids absorbed by the product and to prevent the wetted product from leaking. Therefore, the backsheet should be fluid-impermeable at least in the area where the absorbent core is located. However, the backsheet may allow passage of gases such as water vapor.

The backsheet can be made of a polyethylene (PE) film or a layer of dense nonwoven made of polypropylene (PP) fibres or a laminate of both. The backsheet may be made of a Mater-Bi film offered in the market by Polycart S.p.A. The film in 32 g/m² is suitable as a backsheet of absorbent pads. In a more preferred embodiment, the backsheet is made as a fibrous, hydrophobic wet-laid structure offered by Glatfelter company under the trade name Glatpure. The backsheet may comprise a film layer laminated with a nonwoven layer, forming a so-called textile backsheet.

Biodegradable backsheets are also possible. In such embodiments, the backsheet may be or may comprise a sheet of cellulose material or material of a cellulose derivative. The backsheet may be or may comprise a cellulose film such as a cellophane film. The backsheet may contain or may be coated with a softening agent such as glycerol.

In one embodiment, the backsheet comprises or consists of a cellulose film or a film of a cellulose derivative (the former being preferred), laminated with a nonwoven made of cellulose fibers, wherein said film laminated with a nonwoven preferably has a basis weight of 5 to 40 g/m², preferably 10 to 30 g/m², and even more preferably 15 to 25 g/m².

Alternatively, the backsheet may be a sheet made of or comprising cellulose fibrous material, said sheet having pores with a pore size not large enough for water droplet transmission, but large enough for water vapor transmission. Thus, the layer may be breathable. The pore size of said sheet may be smaller than 100 µm, preferably smaller than 50 µm in diameter. In one embodiment, the backsheet does not have relevant pores.

To generate a suitable soft, pleasant feel of the backsheet, plasticizers can be added, such as glycerol. Antioxidants may also be included in the backsheet or film. Desired opacity of the backsheet can be achieved by addition of neutral, mineral fillers, such as talc, calcium carbonate or silica.

Such technology is described in "The Plasticization Effect of Glycerol and Water on the gelatinization of Wheat Starch by Gena Nashed et al. (April 2003, Starch - Stärke 55 (3-4), DOI: 10.1002/star.200390027). Another way to obtain such films is described in "Novel Starch/Chitosan/Aloe Vera Composites as Promising Biopackaging Materials" by Dagmara Baier et al., J. Polym. Environ 28, 1021-1039 (2020). Xu et al. describe in "Robust and Flexible Films from 100% Starch Cross-Linked by Biobased Disaccharide Derivative" the use of oxidized sucrose for crosslinking the starch (ACS Sustainable Chem. Eng. 2015, 3, 11, 2631-2639). Ning et al. describe the advantage of using citric acid in "Influence of Citric Acid on the Properties of Glycerol-Plasticized Cornstarch Extrusion Blends" (School of Science, Tianjin University, China, in Polymers & Polymer Composites, Vol. 15, No. 7, 2007).

A fibrous sheet as a backsheet can be produced by wet laid technology in which a suspension of fibers in water will be dewatered on a screen, whereby a fibrous web is formed and remains for further process. Papers, consisting of cellulose pulp fibers, are typically produced by such process. Wet-laid nonwovens can use other, longer fibers, e.g., fibers made of regenerated cellulose like viscose.

Depending on the manufacturing process such nonwovens can be wet-laid or airlaid made. A good description of wet-laid cellulosic nonwovens is given by Hemamalini in "Wet laying nonwovens using natural cellulosic fibers and their blends: process and technical applications. A review" (https://doi.org/10.1080/15440478.2019.1701606). Dry laid nonwovens made of cellulosic fibers are known to those skilled in the art. As an example, dry or air laid nonwovens made of cotton or viscose fibers by carding or Rando process are available in the market.

A viscose-based nonwoven with reduced binder content is described in EP 2138056 A3. A new approach to a viscose-based nonwoven is described in CN2012100077076A. This application covers a spun-laid viscose nonwoven which can be tight enough to provide a barrier function to fluids and a good permeability to water vapor.

A backsheet made of cellulosic fibers and best suitable for the use in this invention has a good breathability, will be non-permeable to fluids like urine and blood under in-use conditions and will preferably be hydrophobic on its inner (facing absorbent core) side.

The backsheet is preferably hydrophobic in nature, as the backsheet should provide a barrier to fluids and forms a container for them. Thus, the backsheet may be coated to provide a desired level of hydrophobicity. A person skilled in the art will appreciate several bio-materials which can be used as a treatment or coating to a wet-laid material and provide a hydrophobicity to it. A method that uses starch as a hydrophobicity (repellency) agent is described in WO2018050317.

Another property that may be desired for a backsheet is softness which provides a pleasant feel for a use and lack of noise when a user moves. Both properties can be improved using a fiber mix containing at least several percent of short cellulosic fibers as well as a mechanical treatment to the sheet. Such treatment can comprise embossing, corrugating and similar deformations of plain web surface. As an example, a soft embossing of a fibrous structure is described in US 6,468,392. While this patent describes mostly hydrophilic fibrous structures, a combination with a hydrophobisation step will result in desired properties for a backsheet. Also, a use of so-called air-through dryer prior to the hydrophobisation step can improve the softness and "silence" of such web. A paper machine equipped with such air-through-dryer is described in US6398916. Also, a ring-rolling technology can be used as described in US 4,517,714.

Other possible materials for the backsheet are poly(butylene succinate co-butylene adipate) known as PBSA polymer which can be obtained from renewable feedstock such as glucose and sucrose. Also, thermoplastic starch (TPS) enhanced by additives and plasticizers can be used for producing a backsheet. A further example of a material usable for producing a backsheet is polyhydroxyalkanoates (PHA).

### Absorbent core

The absorbent core (2) comprises at least the absorbent layer and may further comprise a cover sheet covering the absorbent layer on the side facing the topsheet (23, 21). The absorbent core serves to receive, store and bind body fluids. Typical absorbent layers comprise fibers and particles of superabsorbent polymers. While fibers provide fast fluid intake, its distribution and a certain absorbent core integrity, superabsorbent particles absorb and practically irreversibly store the fluid. Performance parameters of absorbent layers include speed of absorption, absorption capacity, and fluid retention.

The absorbent layer generally comprises a superabsorbent polymer material, such as particles of partly neutralized and partly crosslinked polyacrylic acid. Another possible superabsorbent polymer material is or is based on carboxymethyl cellulose, such as the sodium salt of carboxymethyl cellulose. Also, this superabsorbent polymer material is generally provided in the form of particles. The absorbent layer may comprise particles of superabsorbent polymer and at most 10 % by weight of fibers shorter than 10 mm, preferably shorter 8 mm. Alternatively or additionally, the absorbent layer comprises at least 40 % by weight, preferably at least 50 % by weight of said superabsorbent polymer or particles thereof. An absorbent layer may comprise several sub-layers made of materials providing different properties, e.g. fluid wicking, storing, stain masking etc.

The absorbent layer may be an airlaid composite strip. Composite materials suitable for such an element are offered in the market e.g. by McAirlaid's company under the trade name "SuperCore", e.g., as a T-301-S material consisting of 45% cellulosic pulp fibres and 55% of a superabsorbent polymer. The thickness of this material is, for example, 1.4 mm and its density 214 kG/qm. Another source of such airlaid composite materials is Domtar EAM located in Jesup, GA, USA.

The cover sheet that may be part of the absorbent core is a generally lofty fibrous sheet that may, to some extent, contain superabsorbent polymer particles. The cover sheet is an extensible fibrous sheet. The cover sheet generally comprises fibers and preferably consists essentially of fibers. The cover sheet may consist of fibers and, optionally, superabsorbent polymer particles. The cover sheet can be made of any staple fiber, wherein said fibers may be fibers of PE, PP, polyethylene terephthalate (PET), cellulosic fibers, or mixtures thereof, preferably said fibers are cellulosic fibers. The cellulosic fibers may be cotton fibers, pulp fibers, and/or viscose fibers, preferably cotton fibers. The fibers are preferably laid down by usual textile techniques. The cover sheet may have a basis weight of 20 to 200 g/m², preferably from 30 to 120 g/m², more preferably from 40 to 100 g/m².

### Elastic elements of the hygiene slip

As conventionally known, the slip comprises multiple elastic elements. The slip may include waist elastics (3) that run essentially in cross-direction, generally both in the front panel and in the back panel. The leg elastics (4) essentially run along the leg parts of the slip. The elastics in the front and/or rear panels of the slip (5) are optional. Their function is to further improve the fit of a slip; they generally run in cross-direction, but lower than the waist elastics. The leg cuff elastics (6) form the leg cuffs. The main purpose of the waist elastics (3) is to provide elasticity and fit of the slip to the body of the wearer in the waist area of the wearer. The waist elastics generally narrow the circumference of the slip in the waist area by constricting the elastics over the hips and/or I the waist of the wearer, thus avoiding the sliding-down of the slip when worn. The main purpose of the leg elastics (4) is to provide close fit in the leg area to prevent leakage of fluids. The main purpose of the elastics in the front and/or rear panels of the slip (5) is to provide elasticity and fit in the abdominal and bottom areas of the wearer. The main purpose of the leg cuff elastics (6) is to provide their "standing-up" function, which results in an additional barrier against leakage of body exudates, as described in EP2886090 issued to Dao Paper Corp. or in EP1933798 issued to Procter&Gamble.

Elastics that may be used for these purposes are typical elastic strands as used in disposable hygiene articles; they are generally thin and highly elastic. Elastic threads offered by Lycra under the trade name Spandex (Elastane) may be used in different diameters like in 560 D, 620 D, 720 D or 840 D. Also, elastic polyurethane strips and polyurethane foam strips can be used as the elastics in the hygiene slip of the invention. The design of curved elastics in an absorbent hygiene slip is shown in USD 335707S covering the ornamental design of the product.

The hygiene slip of the invention comprises one or more supportive elastic element(s) (11), and optionally additional supportive elastic elements (12), at least in the front panel of the slip. At least one of said supportive elastic element(s) (11) is attached to the chassis or slip
(i) at first and second waist suspension points (13, 15) in both side-seam areas of the slip, said waist suspension points being not more than 2 cm from the waist edge (19) (measured in longitudinal direction) and
(ii) at a lower suspension point (17) being distanced from the waist edge (19) of the slip (or open form thereof) by at least L/4 of the length (L) of the open form in longitudinal direction and positioned in the middle in cross-direction of the slip.

Both lengths L and L/4 are measured in an open form of the slip when flat on a flat horizontal surface in a state where the open form is not contracted by the action of the elastics. In the production process of the slip, the distances L and U4 may be measured before applying the elastics, so that the open form is in a non-contracted state, from the waist edge to the (planned) lower suspension point.

For measuring distances (L) and U4 in a finished slip, the open form of the slip is produced by cutting the slip on both sides along side-seam edges (31, 32, 33, 34) to open it to its open form. Then, the elastics in the open form of the slip are cut into pieces, e.g. by a scalpel, through the layers of the slip or through the chassis. These cuts may be made every 4 ± 1 mm, measured along each elastic. The cuts are sufficiently near, if the maximum lengths L and U4 are measured when the open form is pressed flat on a flat horizontal surface, and no higher lengths L and U4 are measured by making additional cuts of the elastics.

The slip and its open form have right seam edges (31) and (32) in the front and back panels, respectively, in the hip areas of the wearer. The slip and its open form have left seam edges (33) and (34) in the front and back panels, respectively, in the hip areas of the wearer. The attachment points of the supportive elastics (11) are, on the one hand, the waist suspension points (13, 15) in the side-seam areas, i.e. close to the seam edges, and at the lower suspension point (17). The waist suspension points (13, 15) in the seam areas on the left and right side of the slip or its open form are generally located at most 2 cm from the waist edge (19) in longitudinal direction. Further, the waist suspension points (13, 15) are located preferably at most 2 cm from the respective seam edges. The location of the suspension points has the effect that the lower suspension point as well as abdominal outer tissue (sagging belly) are suspended on the hip of the wearer, exerting force (by the extended elastics) in oblique direction from the lower suspension point in upward and outward direction when the slip is worn. The relative positioning of the suspension points of the supportive elastic elements (11) provides that force is applied by the elastics (11), when in extended form and when the slip is worn, in a minimum angle to the horizontal or cross direction of the slip. The angle may be further increased by further lowering the position of the lower suspension point (17) towards the middle of the slip or chassis in Fig. 1 (towards line C-C in Fig. 1). In this way, oblique abdominal muscles can be stabilized by the slip. The force vector exerted by the extended supportive elastic elements may form an angle with a vector in cross-direction of at least 30°, preferably at least 35°.

At the waist suspension points, the elastics (11) are preferably directly or indirectly connected to the (horizonal) waist elastics (3). The lower suspension point (17) is distanced from the waist edge (19) of the slip by at least ¼ of the length (L) of the open form of the slip in longitudinal direction and positioned in the middle, in cross direction, of the slip or open form. If more than one supportive elastic element from the lower suspension point to each waist suspension point is used in the slip, it is sufficient if one of these fulfils the condition that the lower suspension point (17) is distanced from the waist edge (19) of the slip by at least ¼ of the length (L) of the open form, measured as described above.

The supportive elastic element(s) (11) may be two separate elements, one extending from the right hip attachment point (13) to the lower suspension point (17); the other extending from the left hip attachment point (15) to the lower suspension point (17). Alternatively, the supportive elastic element (11) may be one element extending from the right hip attachment point (13) to the lower suspension point (17) and further to the left hip attachment point (15). In a preferred embodiment, said supportive elastic element(s)(11) connects said waist suspension points (13, 15) with said lower suspension point (17) in a trough-shaped curve.

The supportive elastic element (11) may be strings of round, oval or rectangular cross section. In Fig. 2 and 3, it is depicted with an essentially rectangular cross section that is depicted in Fig. 1 by two parallel lines that together form elastic element (11). The supportive elastic element (11) may consist of one solid strand or ribbon. It may also comprise a group of thinner solid strands or ribbons. In a preferred embodiment, the supportive elastic element (11) has a width of at least 2.5 mm, more preferably at least 5 mm, notably when it has a circular cross-section. The slip may further comprise additional supportive elastic elements (12) that may run essentially parallel to the supportive elastic element (11). However, the main supportive force in oblique direction is generally provided by the supportive elastic element (11).

The supportive elastic elements and the other types of elastics are generally elastic strands and are attached to the material of the chassis by any suitable technical means, preferably by a hotmelt glue of short open time, allowing fast adhesion to the topsheet and/or backsheet. The elastics are generally glued to the topsheet and/or backsheet in extended form, such that the tension under which the elastics are stretched in the moment of glue application remains practically unchanged. This means that the adhesive should prevent the so-called creep.

The hotmelt adhesive may be applied separately for each group of elastics. This means that waist elastics may be applied with a glue provided for attachment of the waist elastics to the chassis. The supportive elastic elements are preferably attached by using a separate glue application. This allows using different glues and different settings (art of nozzle, temperature, amount etc.) for the application of the different types of elastics.

In a preferred embodiment of this invention, the supportive elastic elements are thicker and/or have a higher tension force compared to the waist elastics or those in the front and/or rear panels. The cross section of the supportive elastic elements may be at least 2 times, preferably at least 4 times, more preferably at least 5 times of that of the waist elastics. This allows providing a force high enough to support the sagging belly of the wearer. However, the type of material used (Lycra of polyurethane) are the same as usable for the other types of elastics.

In a preferred embodiment, the post-natal absorbent hygiene slip, comprising a chassis (1) comprising an absorbent core (2), a topsheet (23), and a backsheet (25), and comprising a front panel and a back panel and multiple elastic elements including waist elastics (3), leg elastics (4), elastics in the front and/or rear panels of the slip (5), and leg cuff elastics (6), wherein said slip further comprises one or more supportive elastic element (11) as defined in claim 1.

### Manufacturing method

The hygiene slips of the invention can be manufactured on dedicated production lines. Such lines are offered by different machinery producers worldwide. Zuiko Corporation located in 2-1-2 Saitohanada Ibaraki, Japan and Curt G. Joa Inc. located in Sheboygan Falls, WI, USA, are examples of machinery producers. The principles of the manufacturing process are described in US 9,289,329 and US 8,007,484 both assigned to Curt G. Joa, Also, EP 0405575, WO-2000076444, EP 0437771, and EP 0688551 describe manufacturing technology for such pants. US 6,808,582 describes the technology of application of elastics to the sheets of absorbent hygiene pants. EP 2886341 describes the application of curved elastics.

The process may comprise: (i) providing a sheet for the back sheet (e.g. by unrolling from a roll of said sheet), (ii) applying elastics in extended form on the back sheet, including said supportive elastic element (11) on said back sheet, (iii) placing a sheet for the topsheet thereon and connecting the lateral sides of the top and back sheets, (iv) providing leg openings into the layer structure obtained in the previous step, and (v) cutting open forms of the slips from said layered structure. Thus, application of said supportive elastic element(s) (11), preferably in a trough-shaped curve, is performed before the leg openings are provided. Slips may then be made from the open forms by connecting the side-seam areas as mentioned above.

Since the elastics are applied in extended state thereof, the open forms as released from the production line deforms when the elastics retract to release their tension. In a final step, the seam areas of the open form are connected, e.g. by gluing and/or ultrasound to form the slip of the invention.

## Claims

1. A post-natal absorbent hygiene slip, comprising a chassis (1) comprising a front panel and a back panel and multiple elastic elements including waist elastics (3), leg elastics (4), elastics in the front and/or back panels of the slip (5), and leg cuff elastics (6),
wherein said slip further comprises one or more supportive elastic element (11) at least in the front panel of the slip, said one or more supportive elastic element (11) being attached to the chassis
(i) at first and second waist suspension points (13, 15) in side-seam areas of the slip not more than 2 cm from the waist edge (19) and
(ii) at a lower suspension point (17) being distanced from the waist edge (19) of the slip by at least length L/4 of the length (L) of the open form of the slip in longitudinal direction and positioned in the middle in cross direction of the slip.

2. The slip according to claim 1, wherein said supportive elastic element(s) (11) connects said waist suspension points (13, 15) with said lower suspension point (17) along a trough-shaped curve.

3. The slip according to claim 2, wherein said trough-shaped elastic elements or said slip comprises additional supportive elastic elements (12) positioned above and/or below said supportive elastic element (11).

4. The slip according to any one of claims 1 to 3, comprising an absorbent core (2).

5. The slip according to any one of claims 1 to 4, said chassis comprising a topsheet (23) and a backsheet (25).

6. The slip according to claim 5, wherein the topsheet has, at least in the area covering the absorbent core (2), openings larger than 0.5 mm², preferably larger than 1 mm², more preferably about 2 mm².

7. The slip according to any one of claims 1 to 6, wherein said waist suspension points (13, 15) are located at most 2 cm from the right (31, 32) and left (33, 34) seam edges, respectively.

8. A manufacturing process for producing the slip according to any of claims 1 to 7, **characterized in that** the application of said supportive elastic element (11), preferably in a trough-shaped curve, is performed before the leg openings are provided.
